# EUROPEAN PATENT APPLICATION

(11) **EP 4 840 870 A1**
(43) Date of publication of application: **18.11.2026**
(21) Application number: 25749024.3
(22) Date of filing: 24.01.2025
(51) Int. Cl.: C07H 19/20, C07H 1/06, C12P 19/32

(54) **METHOD FOR PRODUCING DISODIUM-5'-GUANYLATE CRYSTALS**

(30) Priority: 29.01.2024 KR 20240013403
(71) Applicant: CJ Cheiljedang Corporation, Seoul 04560 (KR)
(72) Inventor: KIM, Min Jong, Seoul 04560 (KR); PARK, Young Soo, Seoul 04560 (KR); LEE, Joohang, Seoul 04560 (KR); LIM, Hwayeon, Seoul 04560 (KR); YU, Jae Hun, Seoul 04560 (KR); LEE, Seung-je, Seoul 04560 (KR)
(74) Representative: Meissner Bolte Partnerschaft mbB
(86) International application number: PCT/KR2025/001498
(87) International publication number: WO 2025/165121

(57) **Abstract**

The present disclosure relates to a method for preparing disodium-5'-guanylate crystals, comprising: a first step of preparing a disodium-5'-guanylate seed slurry by mixing a disodium-5'-guanylate solution, a mixed solution of sodium chloride and ethanol, and a disodium-5'-guanylate seed; and a second step of obtaining disodium-5'-guanylate crystals by simultaneously adding a disodium-5'-guanylate solution and ethanol to the disodium-5'-guanylate seed slurry.

## Description

### [Technical Field]

The present disclosure relates to a method for preparing high-purity disodium-5'-guanylate crystals even in the presence of residual impurities in the process solution.

### [Background Art]

In the preparation of fermentation-derived disodium-5'-guanylate heptahydrate, a generally known solvent crystallization method uses a hydrophilic organic solvent. In this process, impurities such as guanosine, guanosine diphosphate (GDP), and guanine are removed by resin column treatment before the final crystallization, after which a hydrophilic solvent is added to proceed with crystallization.

However, this process incurs high costs for subsidiary materials and utilities such as steam due to the large amounts of chemicals and water used in the resin column process, and has the drawback of lagging behind the trend toward eco-friendliness from an ESG perspective. Therefore, process improvements are required to minimize the resin column process in line with the trend toward eco-friendliness.

Accordingly, there is a need for a crystallization method that enables productization in the presence of some impurities such as guanosine diphosphate, which are among key factors that affect the crystallization process, without complete removal thereof by the resin column process.

### [Disclosure]

### [Technical Problem]

An object of the present disclosure is to provide a technique capable of preparing high-purity disodium-5'-guanylate crystals even in the presence of residual impurities in the process solution by omitting a resin column process or reducing impurity removal by a resin column process.

### [Technical Solution]

One aspect of the present disclosure provides a method for preparing high-purity disodium-5'-guanylate crystals.

Another aspect of the present disclosure provides a disodium-5'-guanylate crystal comprising: disodium-5'-guanylate; and guanosine diphosphate in an amount of 0.1 wt.% to 3 wt.% of the total weight of the crystal.

### [Advantageous Effects]

According to the present disclosure, disodium-5'-guanylate crystals of excellent crystal quality may be obtained even in the presence of residual impurities in the process solution.

In addition, according to the present disclosure, disodium-5'-guanylate can be crystallized after partial exclusion or minimal use of a resin column. Therefore, the process is more environmentally friendly, can reduce costs of using a resin column, and can also reduce investment costs for resin column facilities upon investment in new production facilities.

### [Brief Description of Drawings]

FIG. 1 is a flowchart illustrating the method for preparing disodium-5'-guanylate according to the present disclosure.
FIG. 2 is an image of disodium-5'-guanylate crystals prepared according to the example of the present disclosure.
FIG. 3 is an image of disodium-5'-guanylate crystals prepared according to Comparative Example 1 of the present disclosure.
FIG. 4 is an image of disodium-5'-guanylate crystals prepared according to Comparative Example 2 of the present disclosure.
FIG. 5 is an image of disodium-5'-guanylate crystals prepared according to Comparative Example 4 of the present disclosure.

### [Detailed Description of Preferred Embodiments]

The present disclosure will be described in detail as follows. Meanwhile, each description and embodiment described herein can be applied to other descriptions and embodiments, respectively. That is, all combinations of various elements described herein fall within the scope of the present disclosure. Further, the scope of the present disclosure is not limited by the specific description described below.

Furthermore, a person skilled in the art may be able to recognize or identify numerous equivalents to the particular aspects of the present disclosure described herein by using routine experimentation. Moreover, these equivalents are intended to be included in the present disclosure.

As used in the specification and appended claims of the present disclosure, the singular articles ("a", "an", and "the") include plural referents unless the context clearly indicates otherwise. Further, unless the context indicates otherwise, singular terms include their plural forms, and plural terms include their singular forms. As used in the specification and appended claims of the present disclosure, the use of "or" may include the meaning of "and/or", unless indicated otherwise.

As used herein, the term "about" may precede a specific numerical value. As used herein, the term "about" encompasses not only the precise numerical value that is specified after the term but also a range that is approximately or nearly close to that value. Considering the context in which the number is presented, it can be determined whether the specific number mentioned is close to or nearly that number. In one example, the term "about" can refer to a range of -10% to +10% of the numerical value. In another example, the term "about" may refer to a range of -5% to +5% of the given numerical value. However, the term is not limited thereto.

As used herein, terms such as "first, second, third, ...", "i), ii), iii), ...", or "(a), (b), (c), (d), ..." are used to distinguish similar elements and do not imply that the elements are executed continuously or in the listed order. For example, when these terms are used in relation to a method, use, or analytical step, the steps may be performed without any time intervals, simultaneously, or with intervals of several seconds, minutes, hours, days, or months.

As used herein, the term "comprising" refers to the presence of features, steps, or components following the term and does not exclude the presence or addition of one or more other features, steps, or components. As used herein, the components or features following the term "comprising" may be essential or mandatory; however, in certain embodiments, other optional or non-essential components or features may also be included.

The present disclosure relates to a method for preparing disodium-5'-guanylate crystals, wherein a disodium-5'-guanylate solution and ethanol are simultaneously added to a disodium-5'-guanylate seed slurry, thereby enabling the formation of disodium-5'-guanylate crystals in a single step without formation and aggregation of disodium-5'-guanylate amorphous nuclei or phase transition of disodium-5'-guanylate crystals. When disodium-5'-guanylate crystals are formed in two steps involving aggregation of amorphous nuclei and phase transition, there is a possibility that impurities are incorporated during nuclei aggregation. However, in the present disclosure, since disodium-5'-guanylate crystals are formed in a single step, high-purity crystals may be formed even in the presence of impurities in the fermentation broth.

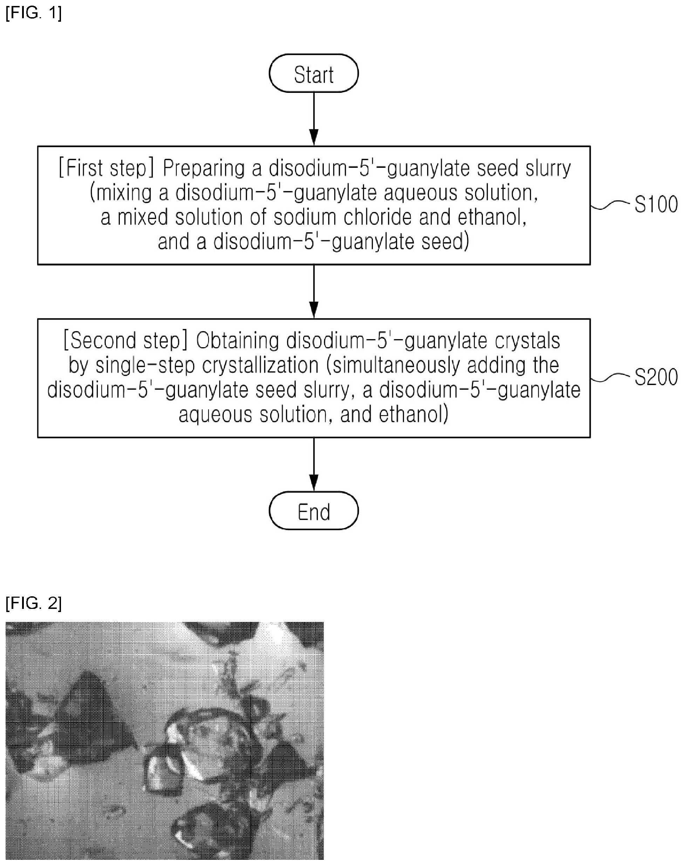

FIG. 1 is a flowchart illustrating the method for preparing disodium-5'-guanylate according to the present disclosure. The method for preparing disodium-5'-guanylate crystals according to FIG. 1 comprises: a first step (S100) of preparing a disodium-5'-guanylate seed slurry by mixing a disodium-5'-guanylate solution, a mixed solution of sodium chloride and ethanol, and a disodium-5'-guanylate seed; and a second step (S200) of obtaining disodium-5'-guanylate crystals by simultaneously adding a disodium-5'-guanylate solution and ethanol to the disodium-5'-guanylate seed slurry. Hereinafter, each step is examined in detail.

In the first step (S100), a disodium-5'-guanylate seed slurry is prepared by mixing a disodium-5'-guanylate solution, a mixed solution of sodium chloride and ethanol, and a disodium-5'-guanylate seed. To this end, in the first step (S100), operations for preparing a disodium-5'-guanylate solution, a mixed solution of sodium chloride and ethanol, and a disodium-5'-guanylate seed, respectively, may be performed.

First, upon examination of the process of preparing a disodium-5'-guanylate solution in the first step (S100), the aqueous solution may be prepared by dissolving disodium-5'-guanylate in a solvent. In particular, the use of organic solvents may be excluded. Disodium-5'-guanylate may be prepared in the form of crude crystals and then dissolved in a solvent.

The disodium-5'-guanylate used in the first step (S100) may be a fermentation product prepared by fermentation. As used herein, the term "fermentation product" may refer to the product resulting from enzymatic or metabolic decomposition of organic substances using microorganisms. For example, a fermentation product may comprise the culture itself obtained by culturing microorganisms in a culture medium, or a concentrate, dried product, or freeze-dried product of the culture obtained after removing the microbial strain therefrom. Moreover, in this case, the fermentation broth may comprise the entirety of the fermentation product comprising disodium-5'-guanylate, or may be a fermentation product comprising disodium-5'-guanylate from which impurities have been removed.

The "microorganism producing disodium-5'-guanylate" or "microorganism producing disodium-5'-guanylate or a target product" used in the first step (S100) includes both wild-type microorganisms and microorganisms that have undergone genetic modification naturally or artificially. It may be a microorganism in which a specific mechanism is weakened or enhanced due to insertion of a foreign gene or enhancement or inactivation of activity of an endogenous gene, and may be a microorganism comprising a genetic modification for production of a protein or disodium-5'-guanylate of interest.

The microorganism producing disodium-5'-guanylate of the present disclosure may be a microorganism naturally having a disodium-5'-guanylate-producing ability, or a microorganism in which a disodium-5'-guanylate-producing ability has been endowed to a parent strain lacking such ability, but is not limited thereto. Specifically, as used herein, a microorganism producing disodium-5'-guanylate or a target product, or a microorganism having a disodium-5'-guanylate- or target product-producing ability, may be a microorganism in which some genes in the biosynthetic pathway of the target protein or target product are enhanced or weakened, or a microorganism in which some genes in the degradation pathway of the target protein or target product are enhanced or weakened. The "enhancement" or "increase" in the disodium-5'-guanylate-producing ability of the microorganism of the present disclosure means that the disodium-5'-guanylate-producing ability of the microorganism of the present disclosure is improved compared to that of microorganisms, parent strains, or unmodified microorganisms excluding the microorganism of the present disclosure. In one example, the microorganism of the present disclosure may have an improved disodium-5'-guanylate-producing ability of at least about 1%, at least 10%, at least 100%, at least 200%, at least 500%, at least 1000%, at least 1100%, at least 1200%, or at least 1300% compared to that of other microorganisms, and may be improved by at least about 1.01-fold, at least 2-fold, at least 5-fold, at least 10-fold, at least 11-fold, at least 12-fold, or at least 13-fold, but is not limited thereto.

The microorganism described above used in the first step (S100) may be at least one selected from the group consisting of the yeast *Candida famata*, the ascomycetes *Eremothecium ashbyii* and *Ashbya gossypii*, the bacterium *Bacillus subtilis*, and microorganisms of *Corynebacterium sp.*

If the microorganism used in the first step (S100) is a microorganism of *Corynebacterium sp.*, the microorganism specifically may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris*, *Corynebacterium crenatum*, or *Corynebacterium flavescens*, and more specifically may be *Corynebacterium glutamicum* or *Corynebacterium stationis*, but is not limited thereto.

The first step (S100) may further comprise culturing a "microorganism producing disodium-5'-guanylate". The culturing of the microorganism may be performed in a suitable medium known in the art under suitable culturing conditions known in the art. Such a culturing process may be readily adjusted and used by those skilled in the art according to the selected strain. Specifically, the culturing may be batch culturing, continuous culturing, or fed-batch culturing, but is not limited thereto. As used herein, the term "medium" refers to a mixed substance containing nutrients required for culturing the microorganism as a main component, and supplies nutrients, growth factors, *etc*., including water, which are essential for survival and development. Specifically, any medium and culture conditions may be used for culturing the microorganism of the present disclosure without particular limitation, as long as the medium is used for the common culture of microorganisms. The microorganism of the present disclosure may be cultured in a common medium that contains suitable carbon sources, nitrogen sources, phosphorus sources, inorganic compounds, amino acids, and/or vitamins, *etc*., while controlling the temperature, pH, *etc*. under aerobic conditions.

In the disodium-5'-guanylate solution, disodium-5'-guanylate may exist in the form of a hydrate. For example, disodium-5'-guanylate may be provided in the form of disodium-5'-guanylate heptahydrate or disodium-5'-guanylate tetrahydrate.

The concentration of disodium-5'-guanylate in the disodium-5'-guanylate solution may be about 150 g/L to about 350 g/L. In some cases, the concentration of disodium-5'-guanylate may be about 200 g/L to about 350 g/L, about 250 g/L to about 350 g/L, about 300 g/L to about 350 g/L, about 150 g/L to about 300 g/L, about 200 g/L to about 300 g/L, about 250 g/L to about 300 g/L, about 150 g/L to about 250 g/L, or about 200 g/L to about 250 g/L. By preparing an aqueous solution of the concentration described above, a yield of disodium-5'-guanylate crystals obtained in the subsequent process can be secured. If the concentration of disodium-5'-guanylate in the aqueous solution is less than about 150 g/L, the yield of disodium-5'-guanylate crystals obtained may decrease due to a low disodium-5'-guanylate content, and if the concentration of disodium-5'-guanylate exceeds about 350 g/L, there is a concern that disodium-5'-guanylate may precipitate before the second step is performed, thereby impeding the process.

The disodium-5'-guanylate solution may be prepared without a process of passing through a resin column to remove impurities. Accordingly, impurities contained in the crude crystals of disodium-5'-guanylate may remain in the disodium-5'-guanylate solution. Specifically, disodium-5'-guanylate analogues such as guanosine, guanosine diphosphate, and guanine may remain in the aqueous solution. In particular, guanosine diphosphate is difficult to separate because it is similar to disodium-5'-guanylate (GMP); however, according to one aspect of the present disclosure, high-purity disodium-5'-guanylate crystals can be obtained without separation of guanosine diphosphate. The concentration of guanosine diphosphate in the aqueous solution may be 5 g/L or less. In some cases, disodium-5'-guanylate may be filtered or decolorized to remove some impurities. However, according to the present disclosure, high-purity disodium-5'-guanylate crystals can be obtained through single-step crystallization without removing impurities contained in disodium-5'-guanylate crude crystals. Therefore, according to one aspect of the present disclosure, by reducing the resin column process used in the conventional process, the use of chemicals and water can be reduced, thereby achieving cost savings and establishing an environmentally friendly process.

The pH of the disodium-5'-guanylate solution may be 8 to 9. In the present disclosure, crystallization can be performed under conditions close to neutral. Therefore, compared to techniques that perform disodium-5'-guanylate crystallization under strongly acidic conditions, there is an advantage in that acid-resistant equipment is not required and the process operation is safe.

In the first step (S100), a step of preparing a mixed solution of sodium chloride and ethanol may subsequently be performed. A mixed solution of sodium chloride and ethanol may be prepared by dissolving sodium chloride in ethanol. Ethanol may be used in an amount corresponding to about 20 vol.% to about 40 vol.% based on the disodium-5'-guanylate solution. Additionally, ethanol may be prepared at a concentration of about 90% and then diluted to about 50% concentration for use. In high-concentration ethanol solutions, the solubility of GMP is very low, such that nuclei of disodium-5'-guanylate are rapidly generated during the crystallization process and the concentration of disodium-5'-guanylate in the solution approaches supersaturation, which may prevent disodium-5'-guanylate crystals from growing. Therefore, by preparing an ethanol solution at the concentration described above, the solubility of disodium-5'-guanylate can be slowly decreased so that the nuclei can grow into crystals.

The concentration of sodium chloride in the mixed solution of sodium chloride and ethanol may be about 5 g/L to 100 g/L. In some cases, the concentration of sodium chloride may be about 10 g/L to about 100 g/L, about 20 g/L to about 100 g/L, about 30 g/L to about 100 g/L, about 40 g/L to about 100 g/L, about 50 g/L to about 100 g/L, about 60 g/L to about 100 g/L, about 70 g/L to about 100 g/L, about 5 g/L to about 80 g/L, about 10 g/L to about 80 g/L, about 20 g/L to about 80 g/L, about 30 g/L to about 80 g/L, about 40 g/L to about 80 g/L, about 50 g/L to about 80 g/L, about 60 g/L to about 80 g/L, about 5 g/L to about 60 g/L, about 10 g/L to about 40 g/L, or about 20 g/L to about 30 g/L.

According to the present disclosure, sodium chloride, which is approved as a food additive, may be added to lower the solubility of disodium-5'-guanylate in hydrophilic solvents. Therefore, the addition of sodium chloride can prevent disodium-5'-guanylate seeds from dissolving in the disodium-5'-guanylate seed slurry. Accordingly, the number of seeds in the seed slurry can be increased, and consequently, the yield of disodium-5'-guanylate crystals obtained in the subsequent crystallization step can be improved. If the concentration of sodium chloride in a mixed solution of sodium chloride and ethanol is less than about 5 g/L, the effect of sodium chloride in preventing the dissolution of disodium-5'-guanylate seeds may be negligible. In addition, if the concentration of sodium chloride in the mixed solution of sodium chloride and ethanol exceeds about 80 g/L, sodium chloride may precipitate in solid form from the ethanol solution. If sodium chloride is precipitated, disodium-5'-guanylate crystallization may not be performed uniformly. This is because precipitated sodium chloride solids may move around in the ethanol solution, causing variations in the composition at different locations within the solution during the crystallization of disodium-5'-guanylate.

With regard to the disodium-5'-guanylate seed, a seed is also referred to as a seed crystal, and may refer to a substance used as a catalyst for the crystallization or granulation of a liquid. Specifically, the seed in the present disclosure may be a crystal of disodium-5'-guanylate.

The disodium-5'-guanylate in the disodium-5'-guanylate seed may be provided in the form of a hydrate. For example, a seed may be provided in the form of disodium-5'-guanylate heptahydrate or disodium-5'-guanylate tetrahydrate. In the first step (S100), disodium-5'-guanylate seeds may be added in a ratio of about 5 wt.% to about 10 wt.% relative to the total weight of the seed slurry. By adding disodium-5'-guanylate seeds in the content range described above, the formation of disodium-5'-guanylate crystals can be optimized.

In the first step (S100), a disodium-5'-guanylate seed slurry is prepared by mixing a disodium-5'-guanylate solution, a mixed solution of sodium chloride and ethanol, and a disodium-5'-guanylate seed prepared as described above. There is no limitation on the mixing method for preparing the seed slurry. For example, the disodium-5'-guanylate solution, the mixed solution of sodium chloride and ethanol, and the disodium-5'-guanylate seed may be mixed using a conventionally used stirring method. There is no limitation on the order in which the disodium-5'-guanylate solution, the mixed solution of sodium chloride and ethanol, and the disodium-5'-guanylate seed are added. For example, the above elements may be introduced sequentially or simultaneously.

The concentration of ethanol in the seed slurry may be 30 wt.% to 50 wt.%. In some cases, the concentration of ethanol may be about 30 wt.% to about 50 wt.%, about 35 wt.% to about 50 wt.%, about 40 wt.% to about 50 wt.%, about 45 wt.% to about 50 wt.%, about 30 wt.% to about 45 wt.%, about 35 wt.% to about 45 wt.%, about 40 wt.% to about 45 wt.%, about 30 wt.% to about 40 wt.%, about 35 wt.% to about 40 wt.%, or about 30 wt.% to about 35 wt.%. The reason the ethanol content in the slurry was adjusted to the range described above is that crystal formation is facilitated when the ethanol in the slurry is provided within the range described above. Furthermore, by matching the ethanol content in the mixed solution at the completion of crystallization in the second step (S200) to a similar level as the ethanol content in the seed slurry prepared in the first step (S100), the effect of changes in solvent content on crystallization during the crystallization process can be minimized.

Next, the second step (S200) is performed to obtain disodium-5'-guanylate crystals by simultaneously adding a disodium-5'-guanylate solution and ethanol to the disodium-5'-guanylate seed slurry.

The disodium-5'-guanylate seed slurry mixed in the second step (S200) is the seed slurry prepared in the preceding first step (S100). In addition, the disodium-5'-guanylate solution mixed in the second step (S200) may also be the solution prepared in the first step (S100). Accordingly, in the first step (S100), a disodium-5'-guanylate solution may be prepared and divided, with some utilized for preparing the seed slurry in the first step (S100) and the rest utilized for performing crystallization in the second step (S200). However, in some cases, for example when performing crystallization in the second step (S200), if a disodium-5'-guanylate solution of a different concentration from that used in the preparation of the seed slurry is required, a disodium-5'-guanylate solution may be prepared again in the second step (S200).

In the second step (S200), ethanol may be added in an amount of 60 vol.% to 80 vol.% relative to the amount of the disodium-5'-guanylate solution added in the second step (S200). In addition, the ethanol may be a solution having an ethanol concentration of 70% to 99.9%. By adding ethanol at the concentration and amount described above in the second step (S200), the ethanol content in the mixed solution at the completion of crystallization in the second step (S200) and the ethanol content in the seed slurry prepared in the first step (S100) may be matched to a similar level. Accordingly, the effect of changes in solvent content on crystallization during the crystallization process can be minimized. Specifically, the concentration of ethanol in the mixed solution obtained by simultaneously adding the disodium-5'-guanylate seed slurry, disodium-5'-guanylate solution, and ethanol is 30 wt.% to 50 wt.%, which may be similar to the ethanol concentration in the seed slurry in the first step (S100) described above.

In the second step (S200), the disodium-5'-guanylate solution and ethanol may be simultaneously added to the disodium-5'-guanylate seed slurry described above. After the above substances are added, disodium-5'-guanylate crystals can be formed in a single step within the reactor without formation and aggregation of disodium-5'-guanylate amorphous nuclei or phase transition of disodium-5'-guanylate crystals.

According to the prior art, when disodium-5'-guanylate in a solution reaches a supersaturation point, nuclei are generated and the nuclei aggregate to form amorphous clusters. Disodium-5'-guanylate hydrate crystals in the form of columnar crystals were prepared through phase transition from amorphous clusters to disodium-5'-guanylate hydrate crystals. The produced disodium-5'-guanylate hydrate crystals in the form of columnar crystals may be disodium-5'-guanylate heptahydrate crystals or disodium-5'-guanylate tetrahydrate crystals. In this process of phase transition from amorphous clusters formed from nuclei aggregation to crystals, various factors such as solvent, temperature, and disodium-5'-guanylate concentration affect the phase transition. In particular, impurities with structures similar to disodium-5'-guanylate, such as guanosine diphosphate, may interfere with the phase transition. As a result, the phase transition to crystals may not occur and the cluster may remain in an amorphous state, or even if phase transition to crystals occurs, the product quality may be significantly degraded. Therefore, according to the prior art, an appropriate crystallization environment must be established in order to crystallize disodium-5'-guanylate hydrate, and in particular, interfering impurities must be removed before crystallization. To this end, impurities such as guanosine, guanosine diphosphate, and guanine were generally removed through a resin column process.

However, according to the present disclosure, by simultaneously adding a disodium-5'-guanylate solution and ethanol to a seed slurry, crystallization proceeds as a single-step process in which seeds within the seed slurry control nucleation and crystals are continuously formed. Therefore, compared to the conventional two-step crystallization method, since there is no amorphous nucleation or aggregation of amorphous nuclei, the present disclosure is relatively free from the issues of impurities becoming trapped between nuclei to form impurity-containing crystals, or impurities limiting crystal growth. Accordingly, disodium-5'-guanylate crystals of excellent crystal quality can be obtained even in the presence of residual impurities in the process solution.

A step of separating the disodium-5'-guanylate crystals obtained after performing the second step (S200) from the mother liquor and drying the same may additionally be performed. The step of separating and drying the crystals can be performed using conventional methods. For example, solid-liquid separators such as a reduced-pressure membrane filtration device, pressure-driven membrane filtration device, or a centrifugal separation device may be used for separation of the crystals, but are not limited thereto.

In the second step (S200), the crystallization process can be performed at a temperature of about 30°C to about 50°C. Uniform crystallization of disodium-5'-guanylate can be achieved within the temperature range described above. The crystallization process can be performed for about 4 hours to about 10 hours.

The crystals prepared after the second step (S200) may be disodium-5'-guanylate hydrate crystals, specifically disodium-5'-guanylate heptahydrate crystals or disodium-5'-guanylate tetrahydrate crystals.

Guanosine diphosphate may remain in the disodium-5'-guanylate crystals prepared after the second step (S200). Guanosine diphosphate may remain in the crystals at a ratio of about 0.1 wt.% to about 3 wt.% of the total weight of the crystals. However, compared to the conventional crystallization method in which phase transition occurs after aggregation of amorphous nuclei, the crystallization method according to the present disclosure has less impact on crystallization even in the presence of residual guanosine diphosphate. Therefore, the morphology, size, purity, and moisture content of the crystals can satisfy the desired product quality even in the presence of residual guanosine diphosphate in the crystals.

Next, according to one aspect of the present disclosure, a disodium-5'-guanylate crystal comprising: disodium-5'-guanylate; and guanosine diphosphate in an amount of 0.1 wt.% to 3 wt.% of the total weight of the crystal is provided.

According to the present disclosure, disodium-5'-guanylate crystals can be prepared through a single-step process as reviewed above, and disodium-5'-guanylate crystals of excellent crystal quality can be obtained even in the presence of residual impurities in the process solution. In general, impurities such as guanosine, guanosine diphosphate, and guanine remain in the crystallization solution for preparing disodium-5'-guanylate crystals, and since these impurities have physical properties similar to those of disodium-5'-guanylate, they are difficult to separate from the process solution.

By utilizing the process according to the present disclosure, disodium-5'-guanylate crystals can be prepared even when the difficult-to-separate impurities described above remain, particularly guanosine diphosphate. The disodium-5'-guanylate crystals prepared in this way provide guanosine diphosphate in an amount of 0.1 wt.% to 3 wt.% of the total weight of the crystals.

The content of guanosine diphosphate described above cannot be exhibited by disodium-5'-guanylate crystals prepared through the two-step process of nucleation and crystal phase transition according to the prior art. As will be examined in the experimental examples below, this is because when the two-step process according to the prior art is performed, it is difficult to obtain crystals even when the guanosine diphosphate concentration is as low as 0.9 wt.%. In addition, when a resin column process is performed prior to obtaining the crystals as in the prior art, guanosine diphosphate in the process solution is almost completely removed, and therefore, the guanosine diphosphate concentration in the crystals likewise cannot be 0.1 wt.% to 3 wt.%.

Furthermore, the disodium-5'-guanylate crystals described above may exhibit an elliptical form.

Moreover, the disodium-5'-guanylate crystals described above may be crystals in a hydrate form, and in some cases may be disodium-5'-guanylate heptahydrate crystals or disodium-5'-guanylate tetrahydrate crystals.

Accordingly, according to one aspect of the present disclosure, a disodium-5'-guanylate crystal comprising: disodium-5'-guanylate; and guanosine diphosphate in an amount of 0.1 wt.% to 3 wt.% of the total weight of the crystal is provided. Since the crystals have an excellent crystal morphology despite containing impurities, they are easy to separate, and since they have a high disodium-5'-guanylate content, they are suitable for use.

### [Mode for Carrying Out the Invention]

The present disclosure will be described in detail by way of Examples. However, these Examples are given for illustrative purposes only, and the scope of the present disclosure is not intended to be limited by these Examples. Meanwhile, technical descriptions absent in the present disclosure may be sufficiently understood and readily practiced by those skilled in the art of the present disclosure or related art.

A method for preparing disodium-5'-guanylate (GMP) crystals according to one aspect of the present disclosure has been examined. Hereinafter, the advantageous effects mentioned in the present disclosure will be examined through the experimental results of Examples and Comparative Examples.

### Example 1. Preparation of disodium-5'-guanylate crystals by single-step crystallization

To evaluate the feasibility of the disodium-5'-guanylate crystallization method according to the present disclosure in the presence of residual guanosine diphosphate in the fermentation broth, disodium-5'-guanylate crude crystals of 90% purity derived from microbial fermentation broth were dissolved to a concentration of 260 g/L, and guanosine diphosphate was artificially added to adjust the guanosine diphosphate content to 0.9 wt.% relative to the mass of disodium-5'-guanylate.

90% ethanol corresponding to 30 vol.% of this solution was diluted to 50% ethanol, sodium chloride was dissolved in the diluted ethanol to 80 g/L, and then 10 wt.% of disodium-5'-guanylate seeds were added to prepare a seed slurry.

To this seed slurry, a disodium-5'-guanylate solution and 90% ethanol at 80 vol.% relative to the disodium-5'-guanylate solution were simultaneously added to continuously generate crystals and perform crystallization.

The crystallization process was performed at 42°C for 6 hours. Thereafter, the crystals and mother liquor were separated using a centrifuge after cooling to 25°C, and the recovered crystals were dried.

The obtained crystals were confirmed to have a disodium-5'-guanylate content of 99.1% and a moisture content of 23.0%, confirming that they are of quality suitable for commercialization. It was confirmed that 0.3% of guanosine diphosphate remained in the prepared disodium-5'-guanylate crystals.

In particular, the disodium-5'-guanylate content can be calculated as follows. 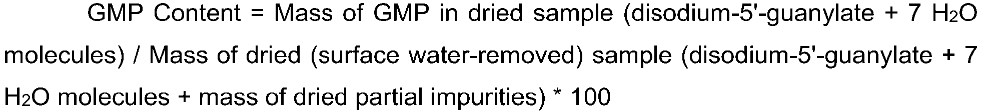 GMP Content = Mass of GMP in dried sample (disodium-5'-guanylate + 7 H2O molecules) / Mass of dried (surface water-removed) sample (disodium-5'-guanylate + 7 H2O molecules + mass of dried partial impurities) * 100

The final product of disodium-5'-guanylate is in the form of a heptahydrate product, in which seven H₂O molecules are present within the crystal. In addition, since surface water is present on the crystal surface before drying, the content of the final product can be expressed as the content of disodium-5'-guanylate (including water of crystallization) present in the total mass of the product with surface water removed by drying. With reference to FIG. 2, it can be confirmed that the obtained disodium-5'-guanylate crystals exhibit an elliptical form rather than a columnar form.

### Comparative Example 1 Preparation of Disodium-5'-Guanylate Crystals Using Nucleation and Crystal Phase Transition (GDP Concentration of 0.1 wt.% or Less)

Disodium-5'-guanylate crude crystals of about 90% purity derived from microbial fermentation broth were dissolved to a concentration of 260 g/L. Guanosine diphosphate was present in the solution at 0.1 wt.% or less relative to the mass of disodium-5'-guanylate.

This solution was placed in a crystallization vessel, the temperature was maintained at 34°C, and 90% ethanol at 110 vol.% relative to the dissolved solution was added over 6 hours. Upon addition of ethanol, disodium-5'-guanylate nucleation and aggregation occur, resulting in precipitation of disodium-5'-guanylate amorphous crystals.

If 10 wt.% of disodium-5'-guanylate seeds are added about 1 hour after the addition of ethanol and ethanol is continuously added, disodium-5'-guanylate crystallization proceeds through additional disodium-5'-guanylate nucleation, formation of disodium-5'-guanylate amorphous crystals by aggregation of previously generated nuclei, and phase transition of amorphous crystals to columnar crystals.

Upon completion of crystallization, the disodium-5'-guanylate crystals were cooled to 25°C, the crystals and mother liquor were separated using a centrifuge to recover the crystals, and the crystals were dried at room temperature to obtain the final product.

The quality of the obtained product was confirmed to have a disodium-5'-guanylate content of 101.1% and a moisture content of 19.3%, and guanosine diphosphate content in the crystals was confirmed to be 0%.

As used herein, disodium-5'-guanylate content refers to the ratio of the mass of disodium-5'-guanylate to the total mass of the sample excluding moisture (mass of disodium-5'-guanylate including moisture / mass of dried sample (excluding moisture) * 100).

Furthermore, moisture content refers to the ratio of the mass of moisture, including water of crystallization and surface moisture, to the mass of solid substance of the sample.

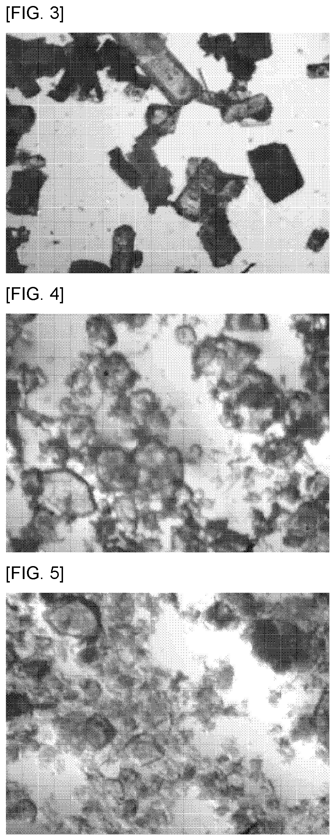

As can be confirmed in FIG. 3, the morphology of the obtained crystals was columnar.

Therefore, from the experiment of Comparative Example 1, it was confirmed that the two-stage crystallization process (amorphous nucleation and phase transition to columnar form) can be performed normally when the guanosine diphosphate concentration in the process solution is 0.1 wt.% or less.

### Comparative Example 2 Preparation of Disodium-5'-Guanylate Crystals Using Nucleation and Crystal Phase Transition (GDP Concentration of about 0.5 wt.%)

Disodium-5'-guanylate crude crystals of about 90% purity derived from microbial fermentation broth were dissolved to a concentration of 275 g/L, and guanosine diphosphate was added to adjust the guanosine diphosphate content to 0.5 wt.% relative to the mass of disodium-5'-guanylate.

This solution was placed in a crystallization vessel, the temperature was maintained at 34°C, and 90% ethanol at 110 vol.% relative to the dissolved solution was added over 6 hours. 10 wt.% of disodium-5'-guanylate seeds were added about 1 hour after adding ethanol, and ethanol was added for the remaining 5 hours to proceed with crystallization.

Upon completion of crystallization, the disodium-5'-guanylate crystals were cooled to 25°C, the crystals and mother liquor were separated using a centrifuge to recover the crystals, and the crystals were dried at room temperature to obtain the final product.

The quality of the product obtained exhibited a disodium-5'-guanylate content of 99.8% and moisture content of 21.0%, which was satisfactory. However, as shown in FIG. 4, the crystal morphology was elliptical rather than columnar, and the guanosine diphosphate content in the crystals was confirmed to be 0.3%.

Therefore, with reference to Comparative Example 2, it was confirmed that disodium-5'-guanylate crystals can also be prepared by using the conventional two-step (amorphous nucleation and phase transition to columnar form) crystallization process. However, as the amount of guanosine diphosphate impurity increased, elliptical crystals rather than columnar crystals were obtained compared to when guanosine diphosphate impurity was present at a very low level of 0.1 wt.% or less (Comparative Example 1), indicating that guanosine diphosphate had an effect on the two-step crystal preparation. Furthermore, it was confirmed that 0.3 wt.%, a considerably large amount of guanosine diphosphate relative to the amount added (0.5 wt.%), remained in the crystals, indicating that a relatively large amount of impurity remained in the crystals during two-step crystallization. This contrasts with Example 1, where only 0.3 wt.% of guanosine diphosphate remained in the crystals even when 0.9 wt.% of guanosine diphosphate was added. Therefore, it can be understood that the two-step disodium-5'-guanylate crystal preparation method is more vulnerable to impurities compared to the crystal preparation method according to Example 1 of the present disclosure.

### Comparative Example 3 Preparation of Disodium-5'-Guanylate Crystals Using Nucleation and Crystal Phase Transition (GDP Concentration of about 0.9 wt.%)

Disodium-5'-guanylate crude crystals of about 90% purity derived from microbial fermentation broth were dissolved to a concentration of 260 g/L, and guanosine diphosphate was added to adjust the guanosine diphosphate content to 0.9 wt.% relative to the mass of disodium-5'-guanylate.

This solution was placed in a crystallization vessel, the temperature was maintained at 34°C, and 90% ethanol at 110 vol.% relative to the dissolved solution was added over 6 hours. 10 wt.% of disodium-5'-guanylate seeds were added 1 hour after adding ethanol, and ethanol was added for the remaining 5 hours to proceed with crystallization.

However, in Comparative Example 3, phase transition to crystals did not proceed properly, such that after disodium-5'-guanylate nuclei precipitated, the crystal morphology remained amorphous and crystal separation was not possible.

Accordingly, it was confirmed that the conventional two-step disodium-5'-guanylate crystal preparation process cannot proceed when the same amount of guanosine diphosphate impurity (0.9 wt.%) as in Example 1 is added. That is, as reviewed above, it can be understood that the conventional two-step disodium-5'-guanylate crystal preparation method is more vulnerable to impurities compared to the crystal preparation method according to Example 1 of the present disclosure.

### Comparative Example 4 Preparation of Disodium-5'-Guanylate Crystals Using Nucleation and Crystal Phase Transition (GDP Concentration of about 1.2 wt.%)

Disodium-5'-guanylate crude crystals of about 90% purity derived from microbial fermentation broth were dissolved to a concentration of 272 g/L, and guanosine diphosphate was artificially added to adjust the guanosine diphosphate content to 1.2 wt.% relative to the mass of disodium-5'-guanylate.

This solution was placed in a crystallization vessel, the temperature was maintained at 34°C, and 90% ethanol at 110 vol.% relative to the dissolved solution was added over 6 hours. 10 wt.% of disodium-5'-guanylate seeds were added about 1 hour after adding ethanol, and ethanol was added for the remaining 5 hours to proceed with crystallization.

Upon completion of crystallization, the disodium-5'-guanylate crystals were cooled to 25°C, the crystals and mother liquor were separated using a centrifuge to recover the crystals, and the crystals were dried at room temperature to obtain the final product.

The product obtained had a disodium-5'-guanylate content of 84.8% and moisture content of 22.0%, with the disodium-5'-guanylate content falling below the quality standard.

As shown in FIG. 5, the morphology of the obtained disodium-5'-guanylate crystals was elliptical, and the guanosine diphosphate content in the crystals was 0.3 wt.%.

As set forth above, those skilled in the art will be able to understand that the present disclosure may be embodied in other specific forms without departing from the technical spirit or essential characteristics thereof. In this regard, it should be understood that the foregoing examples are illustrative in all respects and are not to be construed as limiting. The scope of the present disclosure should be construed as comprising the meaning and scope of the appended claims rather than the detailed description, and all changes or variations derived from the equivalent concepts fall within the scope of the present disclosure.

## Claims

1. A method for preparing disodium-5'-guanylate crystals, comprising:
a first step of preparing a disodium-5'-guanylate seed slurry by mixing a disodium-5'-guanylate solution, a mixed solution of sodium chloride and ethanol, and a disodium-5'-guanylate seed; and
a second step of obtaining disodium-5'-guanylate crystals by simultaneously adding a disodium-5'-guanylate solution and ethanol to the disodium-5'-guanylate seed slurry.

2. The method of claim 1,
wherein the mixed solution of sodium chloride and ethanol comprises sodium chloride at a concentration of 5 g/L to 100 g/L.

3. The method of claim 1,
wherein the concentration of ethanol in the disodium-5'-guanylate seed slurry is 30 wt.% to 50 wt.%.

4. The method of claim 1,
wherein in the second step, the concentration of ethanol in the mixed solution obtained by simultaneously adding a disodium-5'-guanylate solution and ethanol to the disodium-5'-guanylate seed slurry is 30 wt.% to 50 wt.%.

5. The method of claim 1,
wherein in the second step, disodium-5'-guanylate crystals are formed in a single step without formation and aggregation of disodium-5'-guanylate amorphous nuclei or phase transition of disodium-5'-guanylate crystals.

6. The method of claim 1,
further comprising dissolving disodium-5'-guanylate crystals to prepare the disodium-5'-guanylate solution.

7. The method of claim 1,
further comprising separating the disodium-5'-guanylate crystals obtained after performing the second step from the mother liquor and drying the same.

8. The method of claim 1,
wherein the pH of the disodium-5'-guanylate solution is 8 to 9.

9. A disodium-5'-guanylate crystal comprising:
disodium-5'-guanylate; and
guanosine diphosphate (GDP) in an amount of 0.1 wt.% to 3 wt.% of the total weight of the crystal.

10. The disodium-5'-guanylate crystal of claim 9,
wherein the disodium-5'-guanylate crystal exhibits an elliptical form.
